Europäisches Patentamt

(19) **European Patent Office**

**Office européen des brevets**

(11) Numéro de publication: **0 122 201**
**A 1**

# (12) DEMANDE DE BREVET EUROPEEN

(21) Numéro de dépôt: **84400691.6**

(22) Date de dépôt: **06.04.84**

(51) Int. Cl.³: **G 01 N 33/54,** G 01 N 33/58

(30) Priorité: **06.04.83 FR 8305638**

(43) Date de publication de la demande: **17.10.84**
**Bulletin 84/42**

(84) Etats contractants désignés: **AT BE CH DE GB IT LI LU NL SE**

(71) Demandeur: **CENTRE NATIONAL DE LA RECHERCHE SCIENTIFIQUE (C.N.R.S.), 15, Quai Anatole France, F-75700 Paris (FR)**
Demandeur: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Leserman, Lee Daniel, 8, Boulevard de la Rade, F-13007 Marseille (FR)**
Inventeur: **Truneh, Alemseged, Le Grand Large Av. Maurice Germini, F-13260 Cassis (FR)**
Inventeur: **Barbet, Jacques, Rés. Montmorency 73, av. De Lattre de Tassigny, F-13009 Marseille (FR)**
Inventeur: **Machy, Patrick, 11, rue Joinville Provence 4, F-13600 La Ciotat (FR)**

(74) Mandataire: **Gutmann, Ernest et al, Cabinet Plasseraud 84, rue d'Amsterdam, F-75009 Paris (FR)**

(54) **Procédé de détection et de mesure par fluorescence d'une endocytose cellulaire dépendante de protéines de la membrane cellulaire.**

㊗ Procédé de détection et de mesure d'une endocytose cellulaire à la faveur d'une mise en contact avec un ligand déterminé des cellules à étudier, qui comprend la mise en contact de ces cellules et de ce ligand avec des liposomes modifiés par couplage covalent à une molécule elle-même apte à se fixer sur ledit ligand et retenant par encapsulation une solution d'un fluorochrome, caractérisé en ce que la concentration en fluorochrome de la solution encapsulée est initialement suffisamment élevée pour que soit assurée une auto-extinction de sa fluorescence et en ce que l'on mesure ensuite la suppression au moins partielle de l'auto-extinction de la fluorescence qui résulte de l'internalisation desdits liposomes modifiés.

Procédé de détection et de mesure par fluorescence d'une
endocytose cellulaire dépendante de protéines de la
membrane cellulaire.

---

L'invention est relative à un procédé de détection
et de mesure par fluorescence d'une endocytose cellulaire
dépendante de protéines de la membrane des cellules concernées, plus particulièrement à un procédé permettant
des mesures quantitatives de cette endocytose cellulaire.

Il est connu que des molécules de la surface cellulaire appartenant à la catégorie des récepteurs de protéines de transport et d'hormones, des récepteurs Fc
d'immunoglobulines, des immunoglobulines de surface ou
des antigènes codés par le complexe majeur d'histocompatibilité (MHC) peuvent donner lieu à des phénomènes
d'endocytose par les cellules qui les expriment, en
d'autres termes favoriser un mouvement desdites molécules vers l'intérieur desdites cellules ou une pénétration de molécules étrangères dans lesdites cellules, à la
faveur de la réaction desdites molécules avec des molécules capables de les reconnaître spécifiquement.

Les molécules de la susdite catégorie ne sont que
des exemples des nombreux types de protéines ou plus généralement encore des molécules portées par les membranes
cellulaires, notamment à leur surface, et susceptibles de
donner lieu à de tels phénomènes d'endocytose. Pour la
commodité de langage, l'ensemble de ces molécules sera
généralement désigné ci-après par l'expression de "déterminants", en tant qu'ils sont aptes à se combiner avec
une autre classe de molécules ou "ligands", cette combinaison étant alors à l'origine, au moins dans un grand
nombre de cas, de l'induction d'une endocytose cellulaire.

2

Les anticorps monoclonaux constituent une catégorie particulière de ligands, particulièrement indiqués pour l'étude des interactions déterminant-ligand, par exemple en vue de la quantification des déterminants correspondant à la surface des cellules. Cette étude est même facilitée par le fait que l'on peut "visualiser" ces interactions grâce à l'utilisation de liposomes couplés, de façon covalente, à une molécule telle que la protéine A, ces liposomes modifiés pouvant alors être fixés spécifiquement aux déterminants concernés par l'intermédiaire des anticorps monoclonaux correspondants. Lorsque se produit l'endocytose cellulaire, on observe alors une "internalisation" de ces liposomes, en d'autres termes une pénétration de ces liposomes à l'intérieur desdites cellules.

L'utilisation de liposomes ainsi modifiés a déjà été envisagée dans des procédés d'évaluations quantitatives relatives à des réactions d'endocytose mettant en jeu un couple récepteur-ligand déterminé. C'est ce qui a été réalisé, par exemple par P. Machy et Coll. ("Proc. Natl. Acad. Sci. USA", vol. 79, p. 4148-4152, juillet 1982) pour étudier l'endocytose différentielle de déterminants des surfaces de lymphocytes T et B avec des liposomes fluorescents porteurs d'anticorps reconnaissant spécifiquement ces déterminants et contenant du méthotrexate. La fixation des liposomes modifiés à ces déterminants était alors détectée par fluorométrie, tandis que les endocytoses différentielles étaient évaluées par les internalisations différentielles des liposomes modifiés, elles-mêmes mesurées par l'inhibition, médiée par le méthotrexate, de l'incorporation d'une désoxyuridine radioactivement marquée dans les deux types de lymphocytes. Il a alors été constaté que les effets d'inhibition exercés par le méthotrexate transféré à l'intérieur des deux types de lymphocytes,par l'inter-

3

médiaire des liposomes, étaient reconnaissables à la nature desdites cellules et des récepteurs membranaires-cibles correspondants, mais non liés aux nombres respectifs de liposomes fixés à leurs surfaces. Les possibilités d'analyses différentielles auxquelles la méthode décrite donne accès sont cependant encore assez limitées en raison des sensibilités relativement faibles des marqueurs radioactifs, auxquelles s'ajoutent encore les risques liés à la radioactivité.

L'invention a donc pour but de fournir une méthode encore plus sensible - et à l'abri des risques dus à la radioactivité - de détection et de mesure quantitative d'une endocytose cellulaire mettant en jeu un déterminant spécifique des cellules étudiées, à l'occasion de sa reconnaissance par un ligand correspondant.

Elle a encore pour but de fournir une méthode permettant facilement des évaluations quantitatives différentielles des endocytoses induites par la même réaction déterminant-ligand, dans le cas de différents types de cellules susceptibles de porter le même ligand.

Le procédé selon l'invention de détection d'une endocytose cellulaire à la faveur d'une mise en contact avec un ligand déterminé des cellules à étudier et éventuellement porteuses d'un déterminant susceptible d'être reconnu par ledit ligand et avec des liposomes modifiés par couplage covalent à une molécule elle-même apte à se fixer sur ledit ligand - ou au ligand lui-même - et retenant par encapsulation une solution d'un fluorochrome, est caractérisé en ce que la concentration en fluorochrome de la solution encapsulée est initialement suffisamment élevée pour que soit assurée une auto-extinction de sa fluorescence.

Il faut souligner que la susdite mise en contact doit être réalisée à une température à laquelle la cellule est apte à exercer ses fonctions biologiques, par exemple à une température de l'ordre de 37°C pour des cellules d'origine humaine.

4

Il est bien connu que les substances fluorochromes - ou fluorescentes - donnent en général lieu à une auto-extinction très marquée à concentration élevée. Un fluorochrome particulièrement efficace est constitué par la carboxyfluorescéine, qui donne lieu, à concentrations élevées, à une auto-extinction extrêmement forte. Ceci étant, on peut avoir recours à tout autre fluorochrome, choisi de préférence parmi ceux qui se manifestent à l'extinction par un changement de coloration ou analogue, détectable par fluorométrie ou colométrie : on citera par exemple la fluorescéine, l'isothiocyanate de fluorescéine (FITC) ou le fluorochrome dénommé "Lucifer-yellow".

Selon la présente invention, l'activité endocytique est alors détectée et mesurée par la libération dans le cytoplasme du fluorochrome encapsulé dans les liposomes, comme conséquence de l'internalisation des liposomes et de leur transport dans des compartiments intracellulaires, notamment acides. La mesure de l'endocytose est alors directement liée à celle de la diminution de l'extinction de la fluorescence initiale du fluorochrome, donc de l'augmentation, qui peut être quantifiée, de la fluorescence totale associée aux cellules, du fait de la dilution du fluorochrome dans les cytoplasmes de ces cellules.

Ce procédé permet donc de mesurer :
- la densité relative de composants de la surface cellulaire ou à l'inverse d'un ligand susceptible de reconnaître spécifiquement l'un de ces composants cellulaires,

5

- la vitesse à laquelle ces composants peuvent être endocytés.

L'invention offre la possibilité nouvelle et additionnelle de mesurer le devenir du ligand ou du déterminant, c'est-à-dire son endocytose par la cellule qui l'exprime. Cette information peut être obtenue soit pour une population de cellules par fluorométrie conventionnelle ou automatisée, soit pour ces cellules isolées au sein d'une population par cytofluorométrie.

Le procédé selon l'invention est enfin d'une très grande sensibilité. Il permet des analyses différentielles, notamment des mesures différentielles des cinétiques d'internalisation des liposomes, en fonction tant du déterminant ou du récepteur mis en jeu que de la cellule qui l'exprime.

Le procédé selon l'invention permet également de distinguer les couples ligand-déterminant qui, à l'occasion de leur interaction mutuelle, ne conduisent pas à des réactions d'endocytose. Ce sera le cas pour ceux des couples ligand-déterminant qui ne mettent en jeu que des réactions limitées strictement aux surfaces des membranes. Dans un tel cas, la suppression de l'extinction de la fluorescence ne sera pas observée, en raison de l'absence d'internalisation des liposomes.

Les liposomes sont constitués de façon en soi connue. Ils sont essentiellement à base de lipides, notamment de phospholipides, compatibles au moins en partie avec les constituants correspondants des membranes cellulaires. Ils sont préparés par les techniques usuelles, notamment par traitement ultrasonique d'un mélange des lipides choisis et d'une solution concentrée du fluorochrome choisi. Les liposomes formés peuvent ensuite être couplés de façon covalente avec - ou conjugués à - la molécule destinée à assurer la liaison avec le ligand choisi, par exemple la protéine A lorsque le ligand est constitué

6

par un anticorps, par toute méthode permettant ce couplage sans sensiblement affecter les liposomes. Ce couplage est par exemple effectué par l'intermédiaire d'un agent de réticulation, tel que le N-hydroxysuccinimidyl-3-(2-pyridyldithio)-propionate (SPDP), selon les méthodes décrites par L.D. LESERMAN et Coll. ("Nature", vol. 288, n° 5791, p. 602-604, 11 décembre 1980) ou J. BARBET et Coll. ("Journal of Supramolecular Structure and Cellular Biochemistry", 16 : 243-258 (1981), "Cellular Recognition", 237-252).

La molécule permettant la fixation audit ligand et qui est couplée ou conjuguée aux liposomes peut encore être remplacée par le ligand lui-même, le réactif ainsi formé n'étant cependant plus apte qu'à l'utilisation pour la détection de déterminants ou récepteurs correspondants reconnaissables par ce même ligand sur les cellules étudiées.

L'invention met à profit la différence considérable des volumes des liposomes et des cellules à l'étude. Les dimensions des liposomes seront en général inférieures à 4000 Angströms, de préférence inférieures à 1000 Angströms, voire même à 600 Angströms. Si l'on sait que les cellules étudiées originaires de mammifères ont le plus souvent des dimensions de 10 à 15 microns, on reconnaîtra que l'utilisation de liposomes ayant des dimensions de 1000 Angströms (0,1 micron), conduira à un coefficient multiplicateur de volume de l'ordre de $10^6$ qui est à l'origine du phénomène de dilution du fluorochrome libéré à l'intérieur du cytoplasme desdites cellules, à l'occasion de l'internalisation des liposomes dans des cellules subissant l'endocytose à l'occasion de la mise en oeuvre du procédé selon l'invention.

La concentration initiale du fluorochrome des solutions encapsulées est telle que l'on observe de préférence au moins 70 % d'auto-extinction du fluorochrome, de préférence d'au moins 90 % et même 95 % vis-à-vis de l'intensité de fluorescence maximum qui peut être observée pour des solutions plus diluées de ce même fluorochrome. Ces intensités maxima et les concentrations minima permettant d'obtenir les susdits effets d'auto-extinction peuvent être déterminées expérimentalement pour chacun des fluorochromes mis en oeuvre. Dans le cas du fluorochrome préféré, la carboxyfluorescéine, ses concentrations minima seront d'au moins 10 millimolaire, de préférence même d'au moins 100 millimolaire .

Les concentrations de fluorochrome utilisables ne sont pas limitées vers les concentrations les plus élevées, si ce n'est par des considérations de solubilité ou d'ordre pratique. La dilution conduisant à la suppression de l'auto-extinction sera dans tous les cas suffisante, compte tenu des rapports volumiques très élevés entre les liposomes et les cellules étudiées, dont il a déjà été fait état plus haut.

Les mesures relatives à la suppression de l'auto-extinction du fluorochrome mettent en jeu une fonction complexe de plusieurs paramètres, parmi lesquels les trois paramètres suivants :

1°) le taux ou la vitesse d'endocytose du déterminant de surface cellulaire, par conséquent d'internalisation des liposomes ;

2°) la vitesse et le degré de libération de fluorochrome initialement encapsulé par le liposome dans le cytoplasme cellulaire ;

3°) le flux du fluorochrome libéré à l'intérieur de la cellule qui est transporté à l'extérieur de la cellule.

Il résulte de l'examen du premier et du second paramètres que le procédé selon l'invention permet des mesures cinétiques différentielles de l'internalisation

8

des liposomes, par conséquent des évolutions relatives des endocytoses étudiées. Lorsque le troisième paramètre joue un rôle important, il peut limiter les possibilités d'études différentielles de l'endocytose au niveau de la cellule isolée. C'est ce qui peut se produire dans les cas où l'on observerait que le troisième paramètre est prépondérant vis-à-vis du second, c'est-à-dire lorsque le flux de fluorochrome transporté à l'extérieur de la cellule tend à excéder la vitesse ou le degré de libération du fluorochrome initialement encapsulé par le liposome dans le cytoplasme cellulaire. On observera cependant que même dans ce cas, des mesures différentielles pourront être effectuées au niveau global (diminution de l'extinction de fluorescence au niveau de l'ensemble des cellules et du milieu dans lequel elles se trouvent), sinon au niveau de la cellule isolée.

D'autres caractéristiques de l'invention apparaîtront encore au cours de la description qui suit d'exemples de mise en oeuvre du procédé selon l'invention, notamment en rapport avec le dessin dans lequel :

- la fig. 1 est un histogramme de fluorescence établi à partir d'environ 10.000 cellules fibroblastoïdes L de souris marquées selon l'invention et fournissant les intensités relatives de fluorescence (IRF) mesurées à 4°C et 37°C respectivement, en fonction du nombre de cellules (NC),
- la fig. 2 comporte des courbes représentatives de la variation de la fluorescence (F) observée pour un marqueur utilisé dans les conditions de l'invention ou non, pour suivre l'internalisation des liposomes marqués en fonction du temps, en heures (H),
- la fig. 3 est représentative de courbes semblables représentatives des variations de fluorescence observées, selon que le système déterminant-ligand

9

est de ceux qui permettent l'observation d'une endocytose ou au contraire de ceux qui ne le permettent pas.

Dans le cadre du présent exemple, on a utilisé des lymphocytes T provenant de souris CBA/J stimulés par de la concanavaline A et de cellules fibroblastoïdes L. On a effectué des liaisons de conjugués de protéine A et de liposomes aux cellules T et L par l'intermédiaire d'un anticorps monoclonal anti-MHC (H-2) constitué par un anticorps kappa anti-H-2K$^k$ (H100-30/33), du type IgG2b décrit par LEMKE et Coll. (Ed. Hammerling, Elsevier . North Holland Promedical Press, Amsterdam, 102-109, 1981) et par l'intermédiaire d'antisérums de lapins antifibronectine obtenus selon les techniques décrites par RAFF et Coll. (BRAIN RES. 174, 283-308, 1979) et GHANDOUR et Coll. (J. HISTOCHEM. CYTOCHEM., 30, 165-170, 1982). Les conjugués de protéine A et de liposomes ont été préparés comme décrit par LESERMAN et Coll. dans "Nature", 288, 602-604, 1980. Les petits liposomes, obtenus par un traitement aux ultrasons et à base de choline dipalmitoyl-phosphatidyle (65 % molaire), de cholestérol (34 %) et de phosphatidyl-éthanolamine modifié par SPDP (1 %) et contenant de la carboxyfluorescéine 40 mM, ont été incubés pendant 16 heures avec de la protéine A modifiée par SPDP (à raison de 10 moles de SPDP par mole de protéine A) à une concentration finale de protéine A de 200 μg/ml$^{-1}$.

1°) Etude de la liaison spécifique des conjugués de pro téine A et de liposomes aux lymphoblastes T et aux cellules L préincubés avec des anticorps anti-H-2K$^k$ (H100-30/33) ou antifibronectine.

Des cellules T provenant de souris CBA/J sont incubées (30 x 10$^6$ cellules, 37°C, 7 % $CO_2$) dans des flacons de cultures de tissus avec 20 ml de milieu 1 640

10

RPMI (GIBCO) additionné de sérum foetal de veau (FCS) à 5 % inactivé par la chaleur (GIBCO), de glutamine 2 mM, de 2-mercaptoéthanol à 0,05 M de pénicilline, de streptomycine et, afin d'induire la prolifération, de concanavaline A à 2 µg/ml. 40 heures plus tard, on recueille les cellules et on les met en suspension dans un milieu complété sans agent mitotique, à une concentration de $8 \times 10^6$ cellules/ml. Les cellules L mises en culture dans un milieu RPMI additionné de FCS à 7 % sont recueillies par traitement avec de l'EDTA 1,5 mM dans un tampon PBS (5 minutes, 37°C), pour détacher les cellules adhérentes, sont ensuite lavées et remises en suspension à la même concentration. Des aliquotes de 50 µl de cellules sont ensuite placées dans des puits coniques de plaques de microtitrage à 96 puits et 10 µl de H100-30/33 à une concentration finale de 40 µg/ml ou d'antisérums antifibronectine à une dilution finale de 1/100 sont ajoutés aux puits appropriés. Après 1/2 heure d'incubation à 4°C, on lave les cellules une fois et on les remet en suspension dans un milieu frais. 10 µl de conjugué protéine A-liposomes représentant 220 pmoles de carboxyfluorescéine sont ensuite ajoutés à tous les puits contenant des échantillons et on laisse incuber à 4°C, pendant 1,5 heures. De la protéine A libre (80 µg/ml) est ajoutée dans les puits correspondants quelques minutes avant les liposomes. Les cellules sont ensuite lavées trois fois dans un tampon pour liposomes (NaCl : 145 mM ; HEPES 10 mM ; pH 7,4) et la fluorescence associée mesurée sur un spectrofluorimètre Aminco SPF (Excitation : 488 nm, Emission : 520 nm), après lyse des cellules avec du TRITON X-100 à 0,5 %. Les calculs sont faits par référence à des étalons de carboxyfluorescéine à 20 nM.

Les résultats obtenus figurent dans le tableau qui suit :

TABLEAU

| | Anticorps | Protéine-A libre | Carboxyfluorescéine associée à la cellule (pmoles) |
|---|---|---|---|
| Cellules T : | H100-30/33 | − | $4,23 \pm 0,2$ |
| | H100-30/33 | + | $0,06 \pm 0,01$ |
| | − | − | $0,09 \pm 0,01$ |
| Cellules L : | H100-30/33 | − | $7,77 \pm 0,24$ |
| | H100-30/33 | + | $0,07 \pm 0,005$ |
| | anti-fibronectine anti-sérum | − | $1,53 \pm 0,006$ |
| | anti-fibronectine anti-sérum | + | $0,17 \pm 0,05$ |
| | − | − | $0,08 \pm 0,02$ |

Les résultats figurant dans ce tableau témoignent donc de la fixation desdits liposomes sur les cellules.

2°) Etude des cinétiques d'internalisation des liposomes marqués.

Les expérimentations dont les résultats sont illustrés dans les figures ont été réalisées comme suit.

Les lymphoblastes T et les cellules L sont récoltés dans les conditions sus-indiquées, remis en suspension dans un milieu RPMI et additionnés de FCS ($4 \times 10^7$ cellules/ml) et incubés (4°C, 1 heure) dans des tubes à essai en plastique jetables, en présence ou en l'absence d'anticorps. Après un lavage, ils sont réincubés (4°C, 1,5 heures) en présence soit de conjugué protéine A-liposomes (équivalant à 40 µg phospholipide/$10^6$ cellules), soit d'un conjugué FITC-protéine A (20 µg/ml, Pharmacia) et lavés deux fois pour éliminer le matériau non lié. Afin d'examiner la cinétique d'internalisation, on incube les cellules dans un bain d'eau à 37°C, et à des instants différents, on transfère des aliquotes dans des tubes dans un bain de glace, ces tubes contenant un volume de milieu égal à trois fois celui du volume de chacun des aliquotes.

Il en résulte un refroidissement rapide des cellules et l'arrêt des processus enzymatiques et métaboliques dépendant d'un apport en énergie. On analyse ensuite les échantillons sur un détecteur de tri cellulaire EPICS V, en utilisant un modèle laser à ion argon 154 (4 W) . L'excitation laser est de 200 mW à 488 nm avec un filtre Schott SAL 530 en combinaison avec un filtre en verre entaillé ("cut on glass filter") à 530 nm. Le photomultiplicateur est réglé à 600 V. Le flux de cellules est ajusté à 1000 cellules par seconde. Après exclusion des cellules mortes par tri basé sur des mesures de diffraction de lumière, on analyse 10.000 cellules à partir de chaque échantillon pour l'intensité de fluorescence. La carboxyfluorescéine qui n'est pas associée aux cellules n'interfère pas avec les mesures parce que seul un petit volume extra-cellulaire est illuminé.

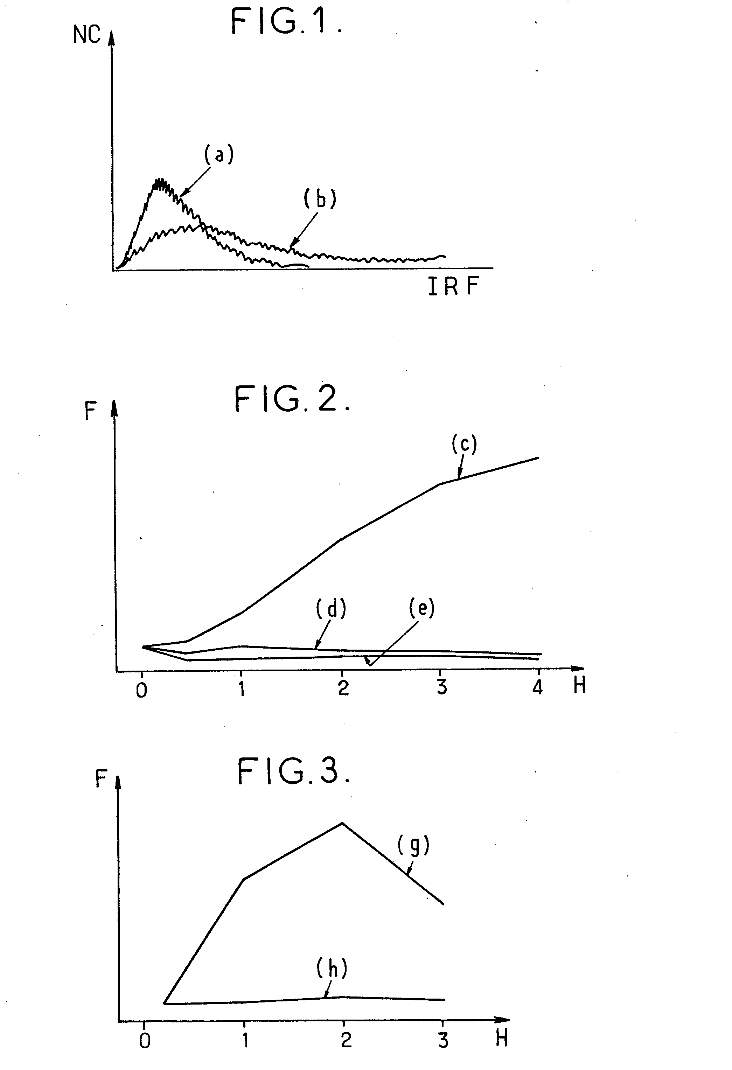

La figure 1 représente un histogramme de fluorescence de cellules L qui ont été colorées avec H100-30/33 (100 µg/ml) et des protéines A-liposomes (CF 40 mM) à 4°C (courbe a) ou après incubation à 37°C (courbe b) pendant 2 heures.

Les variations de fluorescence (F) des cellules T en fonction du temps sont illustrées dans la fig. 2. Les cellules ont été mises en contact soit avec des liposomes contenant de la carboxyfluorescéine 100 mM (courbe c) et 10 mM (courbe d), soit avec un conjugué FITC-protéine A (courbe e). Les valeurs retenues ont été basées sur les fenêtres de fluorescence correspondant à des intensités de fluorescence minima, de façon à exclure 90 % des cellules colorées au temps zéro comme fond de fluorescence. Les intensités de fluorescence, après des temps d'incubation variables à 37°C sont ensuite mesurées et comparées à la mesure initiale.

La fig. 3 fournit des courbes représentatives des variations de fluorescence (F) observées pour des cellules qui ont été incubées en présence des anticorps et en présence des conjugués protéine A-liposome. La courbe g correspond aux observations faites en rapport avec les anticorps H100-30/33 et la courbe h correspond aux observations faites en rapport avec un antisérum-antifibronectine.

Les observations suivantes peuvent être tirées de l'examen du tableau susdit et des figures.

Les lymphocytes T et les cellules L fixent tous deux les conjugués protéine A-liposomes, lorsqu'ils sont incubés à 4°C en présence de H100-30/33, mais pas en son absence. La liaison se produit aussi lorsque les cellules L sont préincubées avec des antisérums antifibronectine. La liaison des conjugués protéine A-liposomes aux deux types de cellules peut être inhibée par les protéines A libres. L'histogramme de fluorescence de la figure effectué sur 10.000 cellules L marquées avec le conjugué protéine A-liposomes par l'intermédiaire des anti-H-2$K^k$ a montré que 98 % de ces cellules avaient été marqués, même si la plupart d'entre elles ne manifestaient qu'une fluorescence faible. Le profil n'est pas modifié de façon importante au bout d'une durée de 4 heures à 4°C (non représenté).

Lorsque ces cellules sont portées à 37°C pendant 2 heures, on observe un déplacement vers les intensités de fluorescence plus élevées (fig. 1). Ce phénomène est lié à la suppression de l'auto-extinction de la carboxyfluorescéine initialement fortement concentrée. Dans l'expérience dont les résultats découlent de la fig. 2, les blastes de cellules T, prétraités par les anticorps monoclonaux anti-H-2$K^k$, ont été incubés à 37°C en présence des deux préparations de conjugués protéine A- liposomes contenant de la carboxyfluoroscéine à des concentrations 100 millimolaire et 10 millimolaire (100 mM et 10 mM), respectivement, mais par ailleurs, identiques en taille, en composition lipidique et en densité de protéine A liée. Ces figures révèlent que les liposomes contenant de la carboxyfluoroscéine 100 mM entraînèrent une augmentation importante de la fluorescéine, celle-ci continuant encore à s'accroître 4 heures après le début des mesures (figure 2). Les cellules incubées avec des liposomes contenant de la carboxyfluoroscéine 10 mM ne présentèrent pas une augmentation de fluorescence analogue. Les valeurs d'auto-extinction pour ces deux types de liposomes étaient initialement de 96 % et 74 % respectivement. Ainsi les liposomes contenant de la carboxyfluoroscéine 100 mM étaient potentiellement aptes à amplifier le signal de fluorescence jusqu'à 25 fois la valeur initiale, lorsqu'est supprimée l'auto-extinction, tandis que les liposomes à 10 mM ne pouvaient amplifier ce signal que 3 à 4 fois.

La fig. 2 montre qu'il n'y a pas eu d'augmentation de la fluorescence lorsqu'on remplace la carboxyfluorescéine non liée dans les liposomes par l'isothiocyanate de fluorescéine (FITC) lié de façon covalente à la protéine A.

La suppression de l'auto-extinction initiale dépend du déterminant de surface cellulaire visé. Une comparaison de cellules L retenant des liposomes par l'intermédiaire d'anticorps contre H-2K$^k$ et d'anticorps contre la fibronectine révèle que seul le déterminant H-2K$^k$ est associé à une augmentation de fluorescence cellulaire (fig. 3), alors que le déterminant fibronectine ne l'est pas. La fibronectine est une protéine structurelle exprimée à la surface des membranes dont on connaissait auparavant l'incapacité à induire une endocytose.

Dans ce qui précède, on a essentiellement pris en considération le cas où les cellules portant le déterminant étudié étaient mises en contact séparément avec le ligand correspondant et les liposomes portant la molécule (ou conjugué à la molécule) susceptible de se lier à son tour à ce ligand.

Les cellules pourraient éventuellement être mises en contact également avec le complexe (liposome-molécule de liaison-ligand) préalablement formé, par exemple avec le complexe liposome-protéine A-anticorps .

Bien que dans ce qui précède, on ait surtout considéré le cas des anticorps, il va de soi que l'invention pourrait être étendue à l'étude des endocytoses cellulaires accompagnant des mises en contact des cellules concernées avec des ligands autres que des anticorps, tels que les hormones ou protéines de transport qui pourraient reconnaître des déterminants correspondants sur lesdites cellules.

D'une façon plus générale encore, le ligand peut être constitué par toute molécule possédant une affinité pour un composant de la membrane cellulaire.

Le procédé selon l'invention est applicable à toute forme de diagnostic in vitro. Par exemple, il pourra être utilisé dans des tests de routine sur des cellules sanguines obtenues, par exemple, par prélèvement veineux, en vue de déterminer les vitesses d'endocytose induites par certains couples ligand-déterminant, en vue de leur comparaison avec les vitesses d'endocytose induites par le même couple dans des cellules d'individus normaux. Dans une autre application, on peut étudier l'effet des principes actifs de médicament à l'égard de cette endocytose, notamment lorsque le principe actif du médicament aura été encapsulé dans les liposomes avec le fluorochrome utilisé. L'action du médicament pourra alors être détectée par les variations constatées au niveau des cinétiques d'internalisation des liposomes marqués, donc des cinétiques de suppressions de l'auto-extinction initiale du flubrochrome.

## REVENDICATIONS

1 -Procédé de détection et de mesure d'une endocytose cellulaire à la faveur d'une mise en contact avec un ligand déterminé des cellules à étudier, qui comprend la mise en contact de ces cellules et de ce ligand avec des liposomes modifiés par couplage covalent à une molécule elle-même apte à se fixer sur ledit ligand et retenant par encapsulation une solution d'un fluorochrome, caractérisé en ce que la concentration en fluorochrome de la solution encapsulée est initialement suffisamment élevée pour que soit assurée une auto-extinction de sa fluorescence et en ce que l'on mesure ensuite la suppression au moins partielle de l'auto-extinction de la fluorescence qui résulte de l'internalisation desdits liposomes modifiés.

2 - Procédé selon la revendication 1, caractérisé en ce que la concentration du fluorochrome est telle que l'auto-extinction observée est égale à au moins 70 % vis-à-vis de la fluorescence maximum qui peut être observée pour des solutions plus diluées du même fluorochrome.

3 - Procédé selon la revendication 2, caractérisé en ce que pourcentage d'auto-extinction est d'au moins 90 %, de préférence d'au moins 95 %,vis-à-vis de l'intensité de fluorescence maximum qui peut être observée pour des solutions plus diluées de ce même fluorochrome.

4 - Procédé selon l'une quelconque des revendications 1 à 3, caractérisé en ce que les liposomes ont des dimensions de préférence inférieures à 4000.Angströms, de préférence inférieures à 1000 Angströms, voire même à 600 Angströms.

5 - Procédé selon l'une quelconque des revendications 1 à 4, caractérisé en ce que le fluorochrome est constitué par la carboxyfluorescéine.

6 - Procédé selon la revendication 5, caractérisé en ce que la concentration de la carboxyfluorescéine encapsulée est au moins 10 millimolaire , de préférence au moins 100 millimolaire .

7 - Procédé selon l'une quelconque des revendications 1 à 6, caractérisé en ce que le ligand est un anticorps monoclonal et la molécule couplée de façon covalente au liposome est de la protéine A.

PLANCHE UNIQUE  0122201

FIG.1.

FIG.2.

FIG.3.

Office européen
des brevets

RAPPORT DE RECHERCHE EUROPEENNE

0122201

Numéro de la demande

EP 84 40 0691

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| D,X<br>Y | NATURE, vol. 288, no. 5791, 11 décembre 1980, pages 602-604, Macmillan Journals Ltd., Chesham, Bucks., GB; L.D. LESERMAN et al.: "Targeting to cells of fluorescent liposomes covalently coupled with monoclonal antibody or protein A"<br>* En entier *<br><br>--- | 1-7 | G 01 N 33/54<br>G 01 N 33/58 |
| Y | NATURE, vol. 293, no. 2829, 17 septembre 1981, pages 226-228, Macmillan Journals Ltd., Chesham, Bucks., GB; L.D. LESERMAN et al.: "Cell-specific drug transfer from liposomes bearing monoclonal antibodies"<br>* En entier *<br><br>--- | 1-7 | |
| D,Y | CHEMICAL ABSTRACTS, vol. 96, no. 7, 15 février 1982, page 447, no. 50315j, Columbus, Ohio, US; J. BARBET et al.: "Monoclonal antibody covalently coupled to liposomes: specific targeting to cells" & J. SUPRAMOL. STRUCT. CELL. BIOCHEM. 1981, 16(3), 243-68<br>* Abrégé *<br><br>---          -/- | 1-7 | **DOMAINES TECHNIQUES RECHERCHES (Int. Cl. ³)**<br><br>G 01 N<br>A 61 K |

Le présent rapport de recherche a été établi pour toutes les revendications

| Lieu de la recherche | Date d'achèvement de la recherche | Examinateur |
|---|---|---|
| LA HAYE | 06-07-1984 | OSBORNE H.H. |

Office européen
des brevets

**RAPPORT DE RECHERCHE EUROPEENNE**

0122201

Numéro de la demande

EP 84 40 0691

| | DOCUMENTS CONSIDERES COMME PERTINENTS | | Page 2 |
|---|---|---|---|

| Catégorie | Citation du document avec indication, en cas de besoin, des parties pertinentes | Revendication concernée | CLASSEMENT DE LA DEMANDE (Int. Cl. ³) |
|---|---|---|---|
| Y | CHEMICAL ABSTRACTS, vol. 93, no. 13, 29 septembre 1980, pages 477-478, no. 130193w, Columbus, Ohio, US; L.D. LESERMAN et al.: "Receptor-modiated endocytesis of antibody-opsonized liposomes by tumor cells" & PROC. NATL. ACAD. SCI. U.S.A. 1980, 77(7), 4089-93 * Abrégé * | 1-7 | |
| Y | CHEMICAL ABSTRACTS, vol. 94, no. 11, 16 mars 1981, page 15, no. 76493z, Columbus, Ohio, US; L.D. LESERMAN et al.: "Specific interaction of myeloma tumor cells with hapten-bearing liposomes containing methotrexate and carboxyfluorescein" & CANCER RES. 1980, 40(12), 4768-74 * Abrégé * | 1-7 | |
| Y | BIOCHIMICA ET BIOPHYSICA ACTA, vol. 551, 1979, pages 295-303, Elsevier/North-Holland Biomedical Press; F.C SZOKA et al.: "The use of aqueous space markers to determine the mechanism of interaction between phospholipid vesicles and cells" * En entier * | 1-7 | DOMAINES TECHNIQUES RECHERCHES (Int. Cl. ³) |

Le présent rapport de recherche a été établi pour toutes les revendications

| Lieu de la recherche LA HAYE | Date d'achèvement de la recherche 06-07-1984 | Examinateur OSBORNE H.H. |
|---|---|---|

OEB Form 1503. 03.82

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant